# EUROPEAN PATENT SPECIFICATION

(11) **EP 1 532 958 B1**
(45) Date of publication and mention of the grant of the patent: **04.03.2009**
(21) Application number: 04027312.0
(22) Date of filing: 17.11.2004
(51) Int. Cl.: A61K 6/08

(54) **Dental surface coating material**
Beschichtungsmaterial für dentale Oberfläche
Produit de revêtement pour une surface dentaire

(30) Priority: 21.11.2003 JP 2003392552
(43) Date of publication of application: 25.05.2005
(73) Proprietor: GC Corporation, Itabashi-ku, Tokyo (JP)
(72) Inventor: Usuki, Daisuke, Itabashi-ku Tokyo (JP); Machida, Daiki, Itabashi-ku Tokyo (JP); Fukushima, Shouichi, Itabashi-ku Tokyo (JP)
(74) Representative: Müller-Boré & Partner Patentanwälte

(56) References cited:
- WO-A-99/17716
- US-A- 5 936 006

## Description

The present invention relates to a dental surface coating material. This material is applied to a surface of a dental material, such as a crown restoration material, a dental filling material, a resin for artificial tooth and denture base or the like, and polymerized by irradiating visible lights to improve surface smoothness and durability of the surface of the dental material.

In a recent dental treatment, a dental material, such as an inorganic material, an organic resin material, an organic and inorganic composite material (it is mainly a dental composite resin) or the like, is widely used to a crown restoration material, a filling material for a damaged part of a tooth, a material for a denture base, or the like. The crown restoration material is, for example, an inlay, an onlay, a crown, a bridge or the like. The filling material is, for example, a dental cement, a dental composite resin or the like. To these dental materials, a carefully polishing work is necessary since a material exposed in an oral cavity is desired to have a smooth surface as much as possible. As the result, there is a problem spending much time for obtaining sufficient surface smoothness. Further, since the dental material is used under severe conditions in the oral cavity, there is also a problem for the material being colored or contaminated due to the deterioration of the surface. For solving these problems, after the surface of the dental material is polished smoothly, the surface is coated with a dental surface coating material having comparatively high hardness, which comprises a polymerizable monomer as a main component.

As a conventional dental surface coating material, an ultraviolet ray-polymerization type dental surface coating material is widely used. This material comprises photopolymerizable oligomer, photopolymerizable monomer, and an ultraviolet-sensitive polymerization initiator and is diverted from an acryl- based hard coating technique widely used in a field of an ultraviolet ray-polymerization type ink or the like. However, the ultraviolet ray-polymerization type dental surface coating material uses an ultraviolet ray having bio-damaging properties at the time of polymerizing, and thus the dental surface coating material using safer visible lights is desired. Then, as a visible light polymerization type dental surface coating material, various composites containing a (meth)acrylate- based monomer and a photopolymerization initiator are proposed.

When the photopolymerizable dental surface coating material comprising the (meth) acrylate- based monomer as the main component is irradiated by visible lights, a radical polymerization chain reaction starts up to generate a polymerization reaction. However, since oxygen in air acts as a polymerization inhibiting factor at this time, an non-polymerized (meth)acrylate- based monomer remains on the surface of the polymerized and cured photopolymerization type dental surface coating material. Therefore, there is a problem that the surface of the cured photopolymerization type dental surface coating material has inferior surface smoothness, or the surface is tacky for a long time without curing in an excessive case. This problem is especially remarkable in the case of the photopolymerization by visible lights rather than that by ultraviolet rays.

For solving this problem, as the various photopolymerization type dental surface coating materials improving the curability of the surface, the following materials are proposed. That is, a visible light polymerization- curable composition comprising a dipentaerythritol acrylate monomer shown with a specific formula, a volatile solvent for the monomer, and a polymerization initiator as main components (for example, with reference to Japanese Patent Laid Open No. 63-183904). A visible light polymerization- curable composition comprising a polyfunctional monomeric acrylate shown with a specific formula, a volatile solvent for the monomer and a thioxanthone photopolymerization initiator as main components (for example, with reference to Japanese Patent Laid Open No. 04-366113). A photopolymerizable dental surface coating material containing a polyfunctional acrylate crosslinking agent produced by modifying 3 or more OH groups in one molecule of pentaerythritol or dipentaerythritol with an acrylic acid ester group, a volatile (meth)acrylate compound, and an acylphosphine oxide for a polymerization initiator (for example, with reference to Japanese Patent Laid Open No. 04-029910). However, these materials have problems that a durability is inferior.

On the other hand, since the strength of the photopolymerizable type dental surface coating material is improved by adding a filling material, large-sized particles, that is, particles having more than about 0.4 µm diameter are generally blended in order to obtain characteristics of high mechanical strength. However, the photopolymerizable type dental surface coating material added with the filling material of such the large-sized particles has problems that glossiness and an aesthetic property of the surface smoothness after polymerizing and curing are inferior, and the durability is also inferior. Therefore, there is a trial that silica particles having 100 nm or less average primary particle diameter are blended as the filling material to have excellent aesthetic property for the surface of the material. However, these silica particles have a shape of an agglomerative powdery silica, and cannot be mono-dispersed in the composite by the conventional technique to exist as the agglomeration. Therefore, the viscosity of the composite remarkably increases, and the composite cannot be used as the photopolymerizable type dental surface coating material. Further, a dental curable material comprising a polymerizable uniform dispersion of a silane- treated silica- urethane (meth)acrylate as a main component is proposed. (for example, with reference to Japanese Patent Laid Open No. 10-218721). In this compound, a silica compound obtained by hydrolyzing a surface of a colloidal silica having 1-85 nm average primary particle diameter with a specific silane compound is uniformly dispersed into urethane (meth)acrylate. In such the photopolymerization type dental surface coating material comprising urethane (meth)acrylate as the main component, the viscosity of the composite is also too high, and thus this material cannot be used as the photopolymerization type dental surface coating material.

Further, US-patent 5,936,006 describes a filled and polymerizable dental material which contains a sol of SiO₂ particles in a liquid, organic dispersion agent, the SiO₂ particles being organically surface modified, having an average size of 10 to 100 nm and being non-agglomerated, and WO-A-99/17716 describes a low-viscosity dental material containing a non-settling nanoscale filler, wherein the filler forms a stable sol with low-viscosity dental materials and may be prepared by surface treatment of fillers having a primary particle size of from about 1 to about 100 nm.

The primary objective of the present invention is to provide a dental photopolymerization type surface coating material, which is fully polymerized by irradiating visible lights to have a smooth surface after curing, and the cured surface has excellent durability and abrasion resistance, when this material is coated on the surface of the dental material to be applied.

The earnest work was carried out by the inventors and, as the result, it was found that the following photopolymerization type dental surface coating material can solve the above-mentioned problems. That is, the material containing 1-70 % by weight of inorganic fine particles having 1-100 nm average particle diameter in a (meth)acrylate monomer in the mono-dispersed state, containing a prescribed amount of a photopolymerization catalyst with respect to the above monomer dispersed with the fine particles, and having 5 Pas (5000cP) or less viscosity at 23°C. This material is rapidly polymerized by visible lights to give excellent surface curability and a coated film has excellent durability and abrasion resistance. A surface of the inorganic fine particle is modified with alkoxysilane having an unsaturated double bond.

In accordance with one aspect of the invention, there is provided a photopolymerization type dental surface coating material, containing 1-70 % by weight of inorganic fine particles having 1-100 nm average particle diameter in a methyl (meth)acrylate monomer and at least one monomer of polyfunctional (meth)acrylate monomers having 4 or more (meth)acryloyl groups in one molecule selected from dipentaerythritol tetra(meth)acrylate, dipentaerythritol hexa(meth)acrylate, dipentaerythritol penta (meth) acrylate, and 1, 3, 5 - tris (1, 3 - bis ( (meth) acryloyloxy)- 2- propoxycarbonylaminohexane)-1,3,5- (1H, 3H, 5H) triazine-2,4,6-trione in the mono-dispersed state, containing 0.01-10 parts by weight of photopolymerization catalyst with respect to 100 parts by weight of above monomers dispersed with the fine particles, and having 5 Pas (5000cP) or less viscosity at 23°C, the surfaces of the inorganic fine particles being modified with alkoxysilane having an unsaturated double bond.

The dental surface coating material according to the present invention can be rapidly polymerized by irradiating visible lights even under the existence of oxygen, and thus can form a strong and transparent cured film having excellent surface curability. This material has excellent surface curability, durability, abrasion resistance and discoloration resistance, in addition to properties of a conventional dental coating composite.

In the present invention, as the inorganic fine particles having 1-100 nm average particle diameter and the surfaces modified with alkoxysilane having an unsaturated double bond, a commonly known compound oxide having the surfaces modified with alkoxysilane can be used without limitation. The compound oxide is, for example, silica, silica-alumina, silica- zirconia or the like. In the dental surface coating material according to the present invention, the inorganic fine particles having 1-100 nm average particle diameter and the surfaces modified with alkoxysilane having an unsaturated double bond is necessary to be blended in the composite in the mono-dispersed state, and thus a material supplied in the mono-dispersed state in the making process of the (meth)acrylate monomer is used. For example, such a material is indicated in Japanese Patent Laid Open No. 07-291817.

As the alkoxysilane having an unsaturated double bond used in the present invention, a silane compound conventionally used in the dental material can be used. More particularly, at least one compound selected from the followings is preferable, that is, 3- methacryloxypropyl trimethoxysilane, 3- methacryloxypropyltriethoxysilane, 3- acryloxypropyltrimethoxysilane, 3- methacryloxy propylmethyldimethoxysilane, 3- methacryloxypropyl methyldiethoxysilane, 3- acryloxypropylmethyl dimethoxy silane, 2- methacryloxyethoxypropyl trimethoxysilane, vinyltrimethoxysilane, vinyltriethoxy silane, and vinyltris(2-methoxyethoxy)silane.

The inorganic fine particles having 1-100 nm average particle diameter and the surface modified with alkoxysilane having an unsaturated double bond, which is used in the present invention, is necessary to be blended 1-70 % by weight in the (meth)acrylate monomer. When the blending amount is less than 1 % by weight, the strength of the surface of the photopolymerization type dental surface coating material after curing is insufficient. When the blending amount is more than 70 % by weight, the viscosity of the photopolymerization type dental surface coating material is too high, and thus it is not preferable. More preferably, the blending amount is 5-30 % by weight.

In the photopolymerization type dental surface coating material according to the present invention, as the (meth)acrylate monomer, which is a base material, containing the inorganic fine particles having 1-100 nm average particle diameter and the surfaces modified with alkoxysilane having an unsaturated double bond, methyl(meth)acrylate and at least one monomer of polyfunctional (meth)acrylate monomers having 4 or more (meth)acryloyl groups in one molecule selected from dipentaerythritol tetra(meth)acrylate, dipentaerythritol hexa(meth)acrylate, dipentaerythritol penta(meth)acrylate, and 1, 3, 5-tris(1, 3-bis((meth)acryloyloxy)-2-propoxycarbonylaminohexane)-1, 3, 5-(1H, 3H, 5H)triazine-2, 4, 6-trione is used. However, when 15-70 % by weight of volatile methyl (meth)acrylate monomer is used in the (meth)acrylate monomer in the whole photopolymerization type dental surface coating material, the volatile (meth)acrylate monomer on the surface of the coated film is rapidly evaporated and then, the concentration of the photopolymerization initiator on the surface increases at the time of coating on the surface of the dental resin material. Then, the surface curability of the photopolymerization type dental surface coating material can increase . Therefore, it is preferable to use the volatile methyl (meth)acrylate monomer.

Methyl methacrylate is the most preferable when safety and volatility are considered. As for the blending amount of the volatile methyl(meth)acrylate monomer used in the present invention, it is preferably 15-70 % by weight in the (meth)acrylate monomer when drying characteristics of the coated surface and the strength after curing are considered. When it is less than 15 % by weight, the drying characteristics and the strength of the surface may decrease. When it is more than 70 % by weight, the surface curability may decrease.

In the photopolymerization type dental surface coating material according to the present invention, the polyfunctional (meth)acrylate monomer having 4 or more (meth) acryloyl groups in one molecule is also used as the (meth)acrylate monomer. The polyfunctional (meth)acrylate monomer having 4 or more (meth)acryloyl groups in one molecule is a monomer having a plurality of (meth) acryloyl groups acting as a cross-linking agent. Further, a monomer having as many polymerization reaction groups as possible is preferably selected in the monomer having the plurality of (meth)acryloyl groups. Then, the polyfunctional (meth)acrylate monomers obtained by modifying 4 or more hydroxyl groups with a (meth)acrylic ester in one molecule are, when operability and safety are considered, selected from dipentaerythritol tetra(meth)acrylate, dipentaerythritol hexa(meth) acrylate, dipentaerythritol penta(meth)acrylate, and 1, 3, 5 - tris (1, 3 - bis ((meth) acryloyloxy)- 2-propoxycarbonylaminohexane)- 1, 3, 5 - (1H, 3H, 5H) triazine- 2,4,6- trione.

As the (meth)acrylate monomer used in the photopolymerization type dental surface coating material according to the present invention, 0.1-10.0 % by weight of (meth)acrylate monomer having an adhesive functional group in the (meth) acrylate monomer can be used. In this case, direct adhesion to a dental cement, a denting, a dental alloy or the like is improved, where the adhesion of these materials is low with respect to the photopolymerization type dental surface coating material. As the (meth)acrylate monomer having the adhesive functional group, the alkoxysilane having an unsaturated double bond, or a (meth) acrylate monomer having an acidic group can be used. As the (meth)acrylate monomer having an acidic group, the following monomers are preferable, that is, a polymerizable monomer or oligomer having an acryloyl group, a methacryloyl group or the like with a carboxyl group, a phosphate group, an acid anhydride residual group, an acid amido group or the like.

As the (meth)acrylate monomer having a carboxyl group, for example, the followings can be used, that is, (meth)acrylic acid, 1,4- di(meth)acryloxyethyl pyromellitic acid, 6- (meth)acryloxyethylnaphthalene 1,2,6- tricarboxylic acid, N,O-di(meth)acryloxytyrosin, O-(meth)acryloxytyrosin, N- (meth)acryloxytyrosin, N-(meth)acryloxyphenylalanine, N- (meth)acryloyl- p-aminobenzoic acid, N- (meth)acryloyl- o- aminobenzoic acid, N- (meth)acryloyl- 5- aminosalicylic acid, N-(meth)acryloyl-4- aminosalicylic acid, 4-(meth)acryloxyethyltrimellitic acid, 4-(meth)acryloxybutyltrimellitic acid, 4-(meth)acryloxyhexyltrimellitic acid, 4-(meth)acryloxydecyltrimellitic acid, 4-(meth)acryloxybutyltrimellitic acid, 2-(meth)acryloyloxybenzoic acid, 3- (meth)acryloyloxy benzoic acid, 4- (meth)acryloyloxybenzoic acid, an addition product of 2- hydroxyethyl(meth)acrylate and maleic anhydride, P- vinylbenzoic acid, O-(meth)acryloxytyrosinamide, N- phenylglycine- glycidyl (meth)acrylate, N- (P- methylphenyl)glycine- glycidyl (meth)acrylate, 11- (meth)acryloxy- 1,1-undecanedicarboxylic acid, 4- [(2- hydroxy- 3- (meth) acryloyloxypropyl)amino] phthalic acid, 5- [(2- hydroxy-3- (meth) acryloyloxypropyl)amino] isophthalic acid, 3-[N- methyl- N- (2- hydroxy- 3-(meth)acryloyloxypropyl)amino] phthalic acid, 4- [N-methyl-N-(2-hydroxy-3-(meth)acryloyloxypropyl)amino] phthalic acid, maleic acid, or the like.

As the (meth) acrylate monomer containing a phosphate group, for example, a (meth)acrylate monomer containing phosphoric acid and a phosphonic acid group is preferable. More particularly, the followings can be used, that is, meth(2- (meth)acryloxyethyl) phosphoric acid, (2- (meth) acryloxyethylphenyl) phosphoric acid, 10-(meth)acryloyloxydecyldihydrogen phosphate, vinyl phosphonic acid, para-vinylbenzylphosphonic acid, or the like. In addition, a (meth)acrylate monomer containing a thiophosphate group can be also used.

As the (meth)acrylate monomer having an acid anhydride residual group, for example, the followings can be used, that is, 4- (meth) acryloxyethyl trimellitic acid anhydride, 6- (meth)acryloxyethylnaphthalene 1,2,6-tricarboxylic acid anhydride, 6- (meth)acryloxyethyl naphthalene 2,3,6- tricarboxylic acid anhydride, 4-(meth)acryloxyethylcarbonylpropanoyl 1,8-naphthalic acid anhydride, 4- (meth)acryloxyethylnaphthalene 1,8-tricarboxylic acid anhydride. The polymerizable compounds having these acid groups may be used by mixing 2 or more kinds.

As for the blending amount of the (meth)acrylate monomer having an adhesive functional group, it is preferably 0.1-10.0 % by weight of the whole (meth)acrylate monomer, and more preferably 0.5-5.0 % by weight. When it is less than 1 % by weight, the adhesion of the photopolymerization type dental surface coating material may decrease. When it is more than 10.0 % by weight, the curability of the surface of the photopolymerization type dental surface coating material may decrease.

In the photopolymerization type dental surface coating material, a photopolymerization type and commonly known polymerization catalyst is used. As the photopolymerization catalyst, a catalyst obtained by combining a sensitizer and a reducing agent is generally used. As the sensitizer, the followings can be used independently or by mixing. That is, camphorquinone, benzil, diacetyl, benzyldimethylketal, benzyldiethyl ketal, benzyl (2 - methoxyethyl) ketal, 4,4'-dimethylbenzyl- dimethylketal, anthraquinone, 1-chloroanthraquinon, 2- chloroanthraquinon, 1,2-benzanthraquinone, 1- hydroxyanthraquinone, 1-methylanthraquinone, 2- ethylanthraquinone, 1-bromo anthraquinone, thioxanthone, 2- isopropylthioxanthone, 2- nitrothioxanthone, 2- methylthioxanthone, 2,4-dimethylthioxanthone, 2,4- diethylthioxanthone, 2,4-diisopropylthioxanthone, 2- chloro- 7- trifluoromethyl thioxanthone, thioxanthone- 10, 10- dioxide, thioxanthone- 10- oxide, benzoin methyl ether, benzoin ethyl ether, isopropyl ether, benzoin isobutyl ether, benzophenone, bis(4- dimethylamino phenyl) ketone, 4,4'-bisdiethylaminobenzophenone, acyl phosphine oxide such as (2,4,6-trimethylbenzoil) diphenylphosphine oxide or the like, or a compound containing an azido group.

As the reducing agent, a tertiary amine is generally used. As the tertiary amine, the followings are preferable, that is, N,N- dimethyl- p- toluidine, N,N-dimethylaminoethylmethacrylate, triethanolamine, 4-dimethylamino methyl benzoate, 4-dimethylamino ethyl benzoate, 4- dimethylamino isoamyl benzoate. Further, as the other reducing agent, the followings can be used, that is, benzoyl peroxide, sodium sulfinate derivative, an organometallic compound or the like.

In the present invention, 0.01-10 parts by weight of photopolymerization catalyst is blended with respect to 100 parts by weight of the sum content of the (meth)acrylate monomer and the inorganic fine particles, which has 1-100 nm average particle diameter and the surface modified with alkoxysilane having an unsaturated double bond, of the photopolymerization type dental surface coating material. When the blending amount is less than 0.01 parts by weight, polymerization curability is inferior. When the blending amount is more than 10 parts by weight, storage stability of the photopolymerization type dental surface coating material decreases.

The photopolymerization type dental surface coating material according to the present invention is needed to have the viscosity of 5 Pas (5000cP) or less at 23°C. If the viscosity is more than 5 Pas (5000cP), the operation for coating on the dental material becomes difficult, the coated film becomes thick, and the strength of the surface after curing decreases. The low viscosity is preferable, and the viscosity of 0,1 Pas (100cP) or less is more preferable.

Further, to the photopolymerization type dental surface coating material according to the present invention, the followings can be suitably blended, that is, a colorant, a polymerization inhibitor, an ultraviolet absorbing agent, an antibacterial agent, a fluorescence agent, a perfume or the like.

### [Example]

A (meth)acrylate monomer sol was prepared by blending inorganic fine particles having 1-100 nm average particle diameter in the mono-dispersed state, and having surfaces modified with alkoxysilane having an unsaturated double bond.

### <Raw Material 1>

A silica-alumina water mono-dispersion sol (Cataloid-SN made by CATALYSTS CHEMICALS IND.CO.,LTD having 12 nm average particle diameter and 20 % by weight silica- alumina concentration) is used as a starting material. A methanol mono-dispersion sol with solid concentration of 20 % by weight was made using this silica-alumina water dispersion sol by substituting the solvent from water to methanol. Then, a mono-dispersion sol, in which the surfaces of the silica- alumina particles are modified with 3 - methacryloxypropyl trimethoxysilane, was made by mixing this methanol mono-dispersion sol and ethanol, and then adding 3- methacryloxypropyl trimethoxysilane to the mixed liquid. Next, the solvent of the obtained mono-dispersion sol was substituted to methylmethacrylate, and a methylmethacrylate mono-dispersion sol of silica- alumina, in which the surfaces were modified with 3- methacryloxypropyl trimethoxysilane, was obtained. This methylmethacrylate mono-dispersion sol of a surface modified silica-alumina has 12 nm average particle diameter of silica- alumina and 20 % by weight concentration, and this sol is said to as "the raw material 1" hereinafter.

### <Raw Material 2>

A methylmethacrylate mono-dispersion sol of a surface-modified silica- alumina was obtained like the raw material 1 except a silica- alumina water dispersion sol having 12 nm average particle diameter and 50 % by weight silica- alumina concentration was used as the starting material in the preparing process of the raw material 1. This methylmethacrylate mono-dispersion sol has 12 nm average particle diameter and 50 % by weight solid concentration, and this sol is said to as "the raw material 2" hereinafter.

### <Raw Material 3>

A methylmethacrylate mono-dispersion sol of a surface-modified silica- alumina was obtained like the raw material 1 except a silica- alumina water dispersion sol having 80 nm average particle diameter and 20 % by weight silica- alumina concentration was used as the starting material in the preparing process of the raw material 1. The methylmethacrylate mono-dispersion sol has 80 nm average particle diameter and 20 % by weight silica-alumina concentration, and this sol is said to as "the raw material 3" hereinafter.

### <Raw Material 4>

A methylmethacrylate mono-dispersion sol of a surface-modified silica was obtained like the raw material 1 except a silica water dispersion sol having 12 nm average particle diameter and 20 % by weight silica concentration was used as the starting material in the preparing process of the raw material 1. The methylmethacrylate mono-dispersion sol has 12 nm average particle diameter and 20 % by weight silica concentration, and this sol is said to as "the raw material 4" hereinafter.

### <Raw Material 5>

A methylmethacrylate mono-dispersion sol of a surface-modified silica- zirconia was obtained like the raw material 1 except a silica- zirconia water dispersion sol having 30 nm average particle diameter and 20 % by weight silica- zirconia concentration was used as the starting material in the preparing process of the raw material 1. In the silica- zirconia water dispersion sol, a weight ratio of SiO₂/ZrO₂ is 3. The methylmethacrylate mono-dispersion sol has 30 nm average particle diameter and 20 % by weight silica- zirconia concentration, and this sol is said to as "the raw material 5" hereinafter.

### Examples 1-13 and Comparison examples 1-6

The blending ratios of examples 1-13 and comparison examples 1-6 were shown in Table 1. 0.5 parts by weight of camphorquinone, 0.25 parts by weight of 4-dimethylamino methyl benzoate and 0.5 parts by weight of (2,4,6- trimethylbenzoil) diphenylphosphine oxide were added to the 100 parts by weight of (meth)acrylate monomers and above raw materials blended as described in Table 1. After mixing, the surface curability, the abrasion resistance and the viscosity of each photopolymerization type dental surface coating material were measured.

### <Evaluation of the surface curability>

The curability measurement was carried out as follows, that is, the method comprising, evenly coating each photopolymerization type dental surface coating material of examples and comparison examples on a disc-like cured body having 15 mm diameter and 1.5 mm height, irradiating lights to the coated body for 20 seconds by a dental light irradiator (LABOLIGHT LV-II, made by GC Corporation) to cure the surface, strongly rubbing the surface of the cured body for 20 seconds by a hard paper (JK WIPER-150-S, made by CRECIA Co., Ltd), and observing surface properties of the body by viewing. The disc-like cured body was made with a glass ionomer cement for filling and repairing (FUJI IILC, made by GC Corporation). The curability was decided as following basis. These results were shown in Table 1 collectively.
× : The surface of the cured body was tacky and had many scratches because it was an unpolymerized polymer.
○: The surface of the cured body was not tacky but has some scratches.
⊚: The surface of the cured body was not tacky and hardly had scratches.

### <Evaluation of the abrasion resistance>

A stainless steel frame having 5 mm overall height was used for making test pieces. The inside of the frame has a shape consisted of a base part having 6 mm diameter and 2 mm height, a test plane having 2.1 mm diameter and 1 mm height, and a truncated conical intermediate part having 2 mm height. This frame was put on a glass board so as to direct the test plane downward. Each photopolymerization type dental surface coating material of examples and comparison examples was charged into the frame by about half, and irradiated by lights for 60 seconds using a visible beam radiator (NEWLIGHT VL-II, made by GC Corporation). Then, the photopolymerization type dental surface coating material was charged to the base plane, and pressure-contacted with the glass board through a cellophane and irradiated by lights for 60 seconds using the visible beam radiator (NEWLIGHT VL-II, made by GC Corporation). After being irradiated more by lights from the test plane for 30 seconds using the visible beam radiator (NEWLIGHT VL-II, made by GC Corporation), the test pieces were removed from a mold and immersed in distilled water at 37°C for 24 hours. The test pieces were mounted to an abrasion testing machine, and reciprocally moved left and right on an emily paper (#600→#1000), to make the base plane and the test plane in parallel each other. The test pieces were once removed and the base parts of the test pieces were coated with a silicone impression material. This test pieces were immersed in a 0.1N- NaOH aqueous solution at 37°C for 6 days. After washing with the distilled water, the height of the test pieces were measured by a micrometer, and were mounted to the abrasion testing machine. A polishing material comprising a spherical powder (250 µm or less) of polymethyl methacrylate and glycerin (1/1(w/V)) was poured on a polishing cloth stuck on the flat glass board. The load to the test piece was set to 8.84kgf/cm². As one right and left reciprocating movement (25 mm sliding distance) was one cycle, the test piece was subjected to a compression sliding movement of 100,000 cycles at 130cycles per minuets. After the test, the height of the test piece was measured. The difference before and after the test was the abrasion loss. These results were shown in Table 1 collectivity.

### <Measuring of the viscosity>

The viscosities were measured at a room temperature (23°C) using a B type rotational viscometer. These results were shown in Table 1 collectivity.

In examples 1-13, all results were preferable. In comparison examples 1-3, the abrasion resistance is low because of not mono-dispersing the inorganic fine particle having 1-100 nm average particle diameter and the surface modified with alkoxysilane having an unsaturated double bond. The comparison example 4 is not suitable as the surface coating material, since these examples did not contain the polymerizable monomer having 4 or more functions and thus, the surface curability is insufficient and the surface is tacky. The comparison examples 5 and 6 are not suitable as the photopolymerization type dental surface coating material, since these examples are filling materials in the not mono-dispersed state and the viscosity is high.

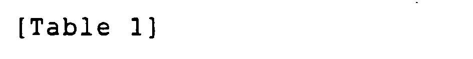

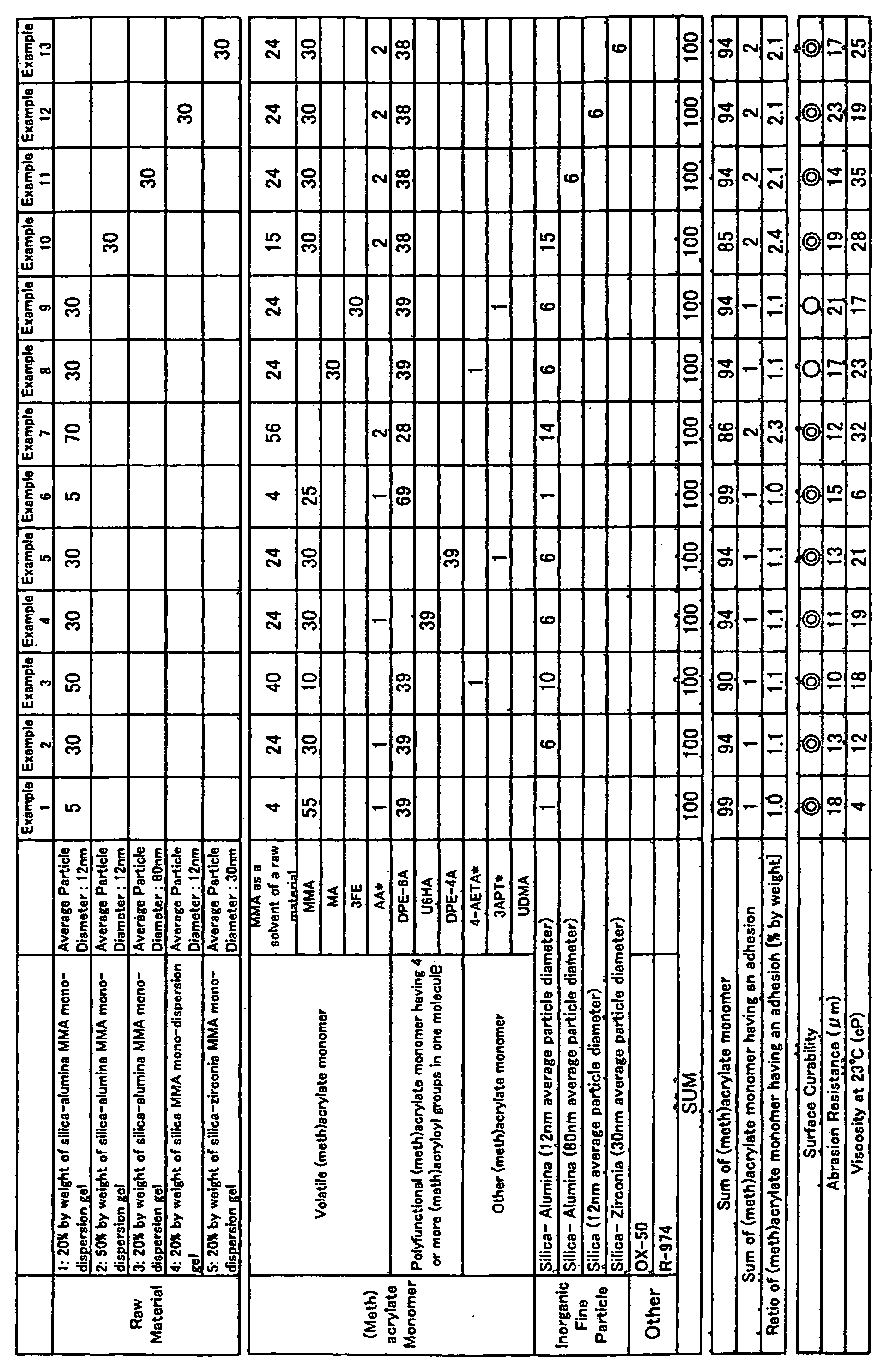

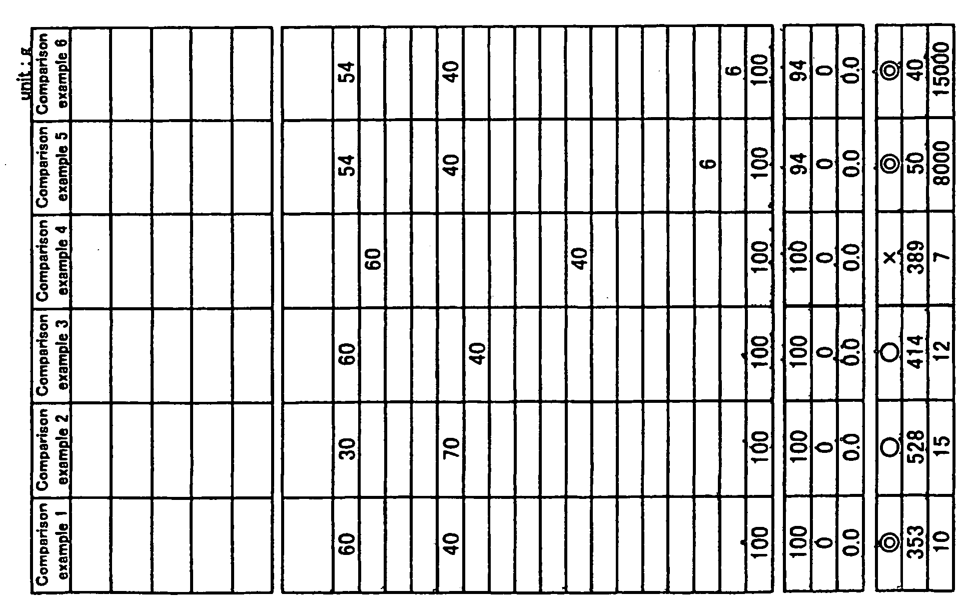

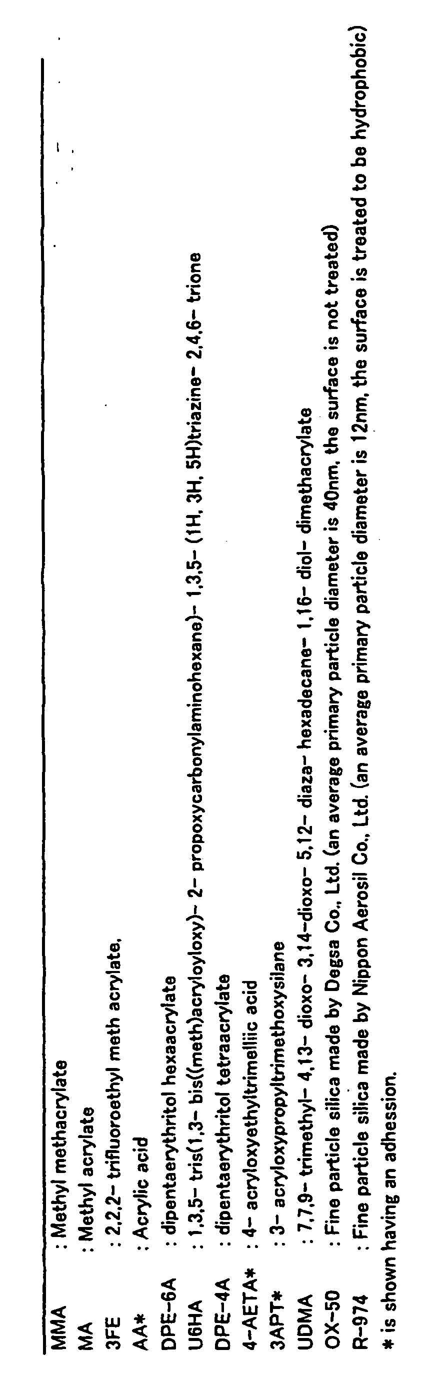

## Claims

1. A dental surface coating material,
wherein 1-70 % by weight of inorganic fine particles having 1-100 nm average particle diameter and surfaces modified with alkoxysilane having an unsaturated double bond is contained in a methyl (meth)acrylate monomer and at least one monomer of polyfunctional (meth)acrylate monomers having 4 or more (meth)acryloyl groups in one molecule selected from dipentaerythritol tetra(meth)acrylate, dipentaerythritol hexa(meth) acrylate, dipentaerythritol penta (meth)acrylate, and 1,3,5- tris (1, 3 - bis ( (meth) acryloyloxy)- 2-propoxycarbonylaminohexane)- 1, 3, 5 - (1H, 3H, 5H) triazine- 2,4,6- trione in the mono-dispersed state, 0.01-10 parts by weight of photopolymerization catalyst is contained with respect to 100 parts by weight of said monomers dispersed with said fine particles, and viscosity is 5 Pa·s (5000cP) or less at 23°C.

## Patentansprüche

1. Beschichtungsmaterial für Dentaloberflächen, wobei
1 bis 70 Gew.-% anorganischer Feinteilchen, die einen mittleren Teilchendurchmesser von 1 bis 100 nm und Oberflächen aufweisen, die mit Alkoxysilan mit einer ungesättigten Doppelbindung modifiziert sind, im monodispersen Zustand in einem Methyl(meth)acrylatmonomer und mindestens einem Monomer von polyfunktionellen (Meth)acrylatmonomeren, die 4 oder mehr (Meth)acryloylgruppen in einem Molekül aufweisen, ausgewählt aus Dipentaerythritoltetra(meth)acrylat, Dipentaerythritolhexa(meth)acrylat, Dipentaerythritolpenta(meth)acrylat und 1,3,5-Tris(1,3-bis((meth)acryloyloxy)-2-propoxycarbonylaminohexan)-1,3,5-(1H,3H,5H)triazin-2,4,6-trion, enthalten sind,
0,01 bis 10 Gewichtsteile eines Photopolymerisationskatalysators, bezogen auf 100 Gewichtsteile des Monomers, das mit den Feinteilchen dispergiert ist, enthalten sind, und
die Viskosität 5 Pa·s (5000 cP) oder weniger bei 23°C ist.

## Revendications

1. Matériau de revêtement de surface dentaire,
dans lequel 1 à 70 % en poids de fines particules minérales présentant un diamètre de particule moyen de 1 à 100 nm, et des surfaces modifiées par de l'alcoxysilane présentant une double liaison insaturée, sont contenus dans un monomère de (méth)acrylate de méthyle, et au moins un monomère parmi des monomères de (méth)acrylate polyfonctionnels présentant 4 ou plus de 4 groupes (méth)acryloyle par molécule, sélectionnés parmi du tétra(méth)acrylate de dipentaérythritol, de l'hexa(méth)acrylate de dipentaérythritol, du penta(méth)acrylate de dipentaérythritol, et de la 1,3,5-tris(1,3-bis((méth)acryloyloxy)-2-propoxycarbonylaminohexane)-1,3,5-(1H,3H,5H)triazine-2,4,6-trione à l'état monodispersé, 0,01 à 10 parts en poids de catalyseur de photopolymérisation étant contenues par rapport à 100 parts en poids desdits monomères dispersés avec lesdites particules fines, et la viscosité étant de 5 Pa·s (5000 cP) ou moins à 23°C.
